# EUROPEAN PATENT APPLICATION

(11) **EP 1 332 770 A1**
(43) Date of publication of application: **06.08.2003**
(21) Application number: 01963518.4
(22) Date of filing: 07.09.2001
(51) Int. Cl.: A61M 25/10, A61M 25/00

(54) **CATHETER FOR TREATMENT OF PROSTATIC HYPERTROPHY**

(71) Applicant: TSUKADA MEDICAL RESEARCH CO., LTD., Tokyo 169-0074 (JP)
(72) Inventor: TSUKADA, Osamu, Ueda-shi, Nagano 386-0002 (JP)
(74) Representative: Makovski, Priscilla Mary
(86) International application number: JP0107786
(87) International publication number: WO03022346

(57) **Abstract**

A catheter 100 for prostatomegaly treatment comprises a catheter body 1 including a perfusion passage 11, a first fluid passage 13, a second fluid passage 14, and an urination passage 15; a branch member 2 integrally formed on a proximal end of the catheter body 1 and having fluid injection and ejection ports communicated to the respective passages; a first balloon 3 adapted to be retained in a bladder, the first balloon 3 being provided on a distal end of the catheter body 1 and communicated to the first fluid passage 13; a second balloon 4 adapted to press a prostate gland, the second balloon 4 being provided on a distal end of the catheter body 1 at the upper stream than the first balloon 3 and communicated to the second fluid passage 14; and a distal end stopper 5 provided on a distal end of the catheter body 1.

## Description

### [TECHNICAL FIELD]

This invention relates to a catheter for prostatomegaly treatment and more particularly relates to a catheter for prostatomegaly treatment to be used for thermotherapy of prostate gland and treatment for post-ablation of a prostate gland.

### [BACKGROUND ART]

The prostatomegaly is a symptom in which a senescent throws a hormone out of balance, nodes are caused in a prostate gland, and the nodes grow and enlarge, thereby pressing a urethra. The prostatomegaly makes urination difficult. Accordingly, various treatments of the prostatomegaly have been adopted. Typical treatments include a pharmacotherapy, an ablation, a thermotherapy, or the like.

The present invention is directed to a catheter to be used in an ablation of prostate gland and a thermotherapy of a prostate gland. The catheter to be used in the ablation of a prostate gland is utilized to exhaust an effusion liquid in a bladder and effect astriction of the prostate gland after removing a part of the prostate gland or when applying a treatment to the prostate gland. The thermotherapy of a prostate gland is a treatment in which abnormal tissues in the prostate gland are heated and atrophied. After applying this treatment to the prostate gland, a catheter is utilized to discharge an effusion liquid from a bladder and perfuse and purge a blood or the like in the bladder. No catheter has been able to effect the perfusion and purgation of the effusion liquid, blood or the like at the same time.

There are two kinds of catheter to be used in ablation treatment of a prostate gland, that is, a double balloon type catheter and a single balloon type catheter. The double balloon type catheter has a series of two discrete balloons on a distal end of the catheter. A leading balloon (5-10 cc in volume) is adapted to be retained in a bladder while a trailing balloon (more than 30 cc in volume) is adapted to press the ablated prostate gland so as to stop bleeding from the gland. The single balloon type catheter omits the balloon to be retained in the bladder and utilizes only the balloon (more than 30 cc in volume) to press the prostate gland so as to stop bleeding from the gland. The single balloon type catheter is once inserted into a bladder at a distal end and drawn back to locate the balloon at a position of a prostate gland. Then, the balloon is inflated to press the prostate gland so as to stop bleeding from it.

A catheter to be utilized in the thermotherapy of prostate gland is a single balloon type catheter having only a balloon to be retained in a bladder. However, according to a recent research, it has been found that the prostate gland immediately after thermotherapy tends to slightly inflate and restore atrophied tissues. Consequently, it has been found to be able to suppress restoration of atrophied tissues of a prostate gland by pressing the tissues after heating by utilizing the double balloon type catheter. However, since the balloon for suppressing the bleeding is designed to be a spherical configuration, the balloon is displaced from the prostate gland or the balloon applies an uneven pressure to the prostate gland when the balloon is inflated. Accordingly, it is impossible to suitably press the prostate gland after heating.

Accordingly, an object of the present invention is to provide a catheter that can press a prostate gland to suppress bleeding and injection and can also purge a perfusion liquid from a bladder simultaneously, thereby allowing the catheter to be shared in the ablation treatment of a prostate gland and the thermotherapy of the prostate gland.

### [DISCLOSURE OF THE INVENTION]

A catheter for prostatomegaly treatment in accordance with the present invention comprises a catheter body including a perfusion passage, a first fluid passage, a second fluid passage, and an urination passage; a branch member integrally formed on a proximal end of the catheter body and having fluid injection and ejection ports communicated to the respective passages; a first balloon adapted to be retained in a bladder, the first balloon being provided on a distal end of the catheter body and communicated to the first fluid passage; a second balloon adapted to press a prostate gland, the second balloon being provided on a distal end of the catheter body at the upper stream than the first balloon and communicated to the second fluid passage; and a distal end stopper provided on a distal end of the catheter body.

A contrast wire may be provided in the catheter body by insert molding, if desired.

In order to form the second balloon for pressing the prostate gland into a substantially cylindrical shape when the second balloon is inflated, a thickness of each of the opposite ends of the second balloon adapted to press the prostate gland may be smaller than that of an intermediate portion of the second balloon or the catheter body may be provided in a plurality of longitudinal positions with outlet ports in the second fluid passage communicated to the second balloon adapted to press the prostate gland.

### [BRIEF DESCRIPTION OF THE DRAWINGS]

Figure 1 is a plan view of a catheter for prostatomegaly treatment in accordance with the present invention;
Figure 2 is a cross sectional view of each part of the catheter shown in Figure 1, (A), (B), (C), and (D) illustrating the respective cross sectional views taken along lines 2A-2A, 2B-2B, 2C-2C, and 2D-2D in Figure 1;
Figure 3 is an enlarged plan view of an end of the catheter shown in Figure 1;
Figure 4 is an enlarged plan view similar to Figure 3, illustration each balloon being inflated;
Figure 5 is a partially enlarged sectional view of a portion of Figure 4; and
Figure 6 is an explanatory view of an example of using the catheter for prostatomegaly treatment in accordance with the present invention.

### [BEST MODE FOR CARRYING OUT THE INVENTION]

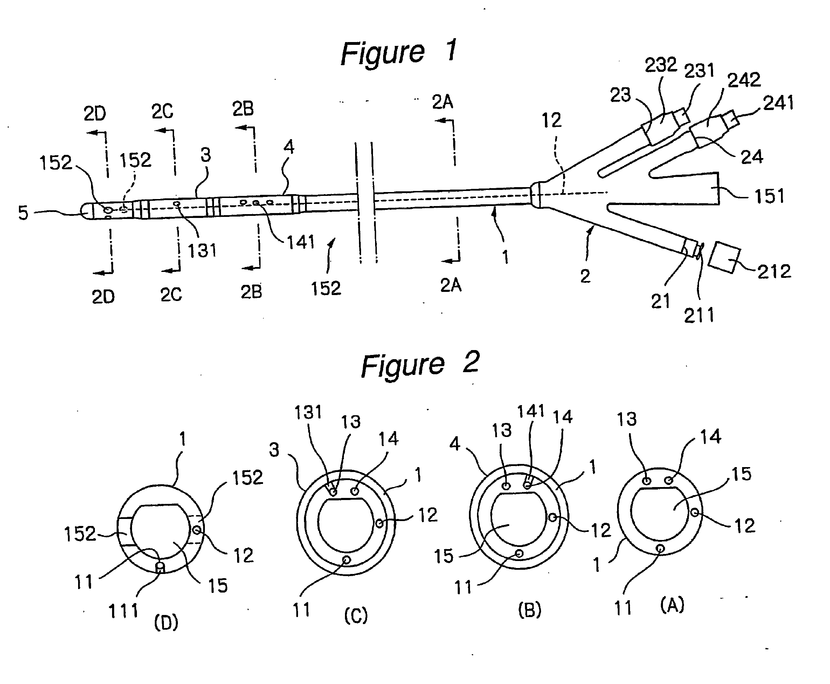

Referring now to Figures 1 through 6, an embodiment of a catheter for prostatomegaly treatment in accordance with the present invention will be described below.

As most clearly shown in Figures 1 and 2, a catheter 100 for prostatomegaly treatment in accordance with the present invention includes generally a catheter body 1, a branch member 2, a first balloon 3, a second balloon 4, and a distal end stopper 5.

The catheter body 1, as most clearly shown in Figure 2, includes a perfusion passage 11, an insert contrast wire 12, a first fluid passage 13, a second fluid passage 14, and an urination passage 15. The insert contrast wire 12 may be embedded in the catheter body 1, if desired. The catheter body 1 is made of silicone rubber, latex, or the like. The insert contrast wire 12 is made of barium sulfate. Since the catheter body 1 made of silicone rubber or the like has lubricative and releasable characteristics, the catheter body 1 is most suitable for a catheter for prostatomegaly treatment of the present invention.

The branch member 2 is formed integrally on a proximal end of the catheter body 1 and has each fluid injection and ejection port communicated to each passage. That is, a perfusion liquid injection port 21 (a female connector 211 and a cap 212 are connected to the port 21) is communicated to the perfusion passage 11; a first fluid injection and ejection port 23 (a one-way valve 231 is connected to the port 23 and a first colored tape (for example, a red tape) is wound around the outer periphery of the port 23 for the purpose of marking) to the first fluid passage 13; a second fluid injection and ejection port 24 (a one-way valve 241 is connected to the port 24 and a second colored tape (for example, a blue tape) is wound around the outer periphery of the port for the purpose of marking) to the second fluid passage 14; and an urination port 151 to the urination passage 15.

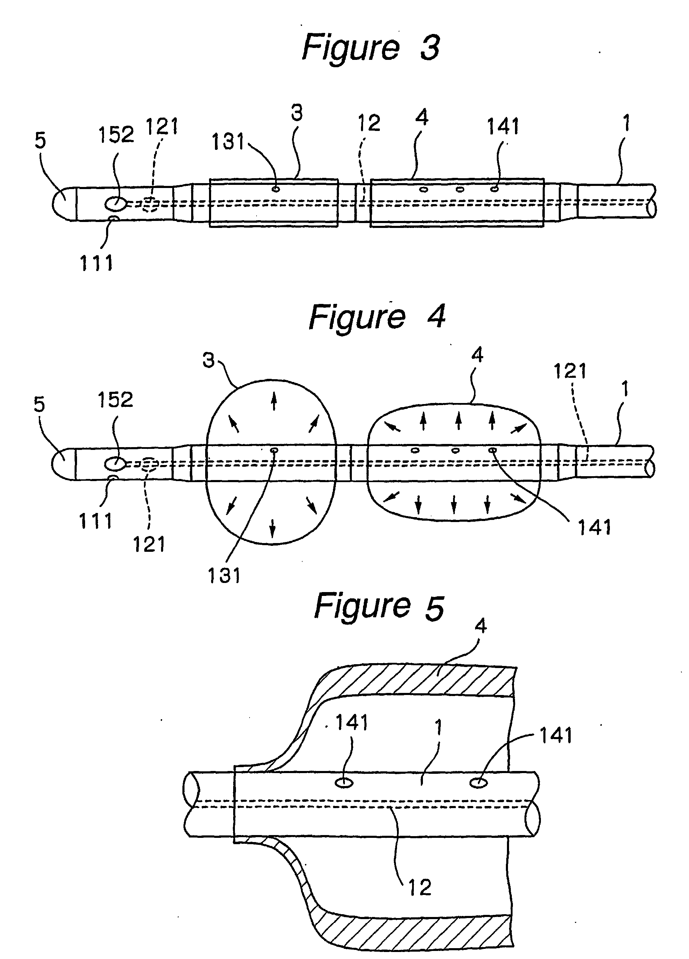

The first balloon 3 to be retained in the bladder, as most clearly shown in Figures 1 and 3, is provided on the distal end of the catheter body 1 and communicated to an outlet port 131 in the first fluid passage 13 (see Figure 2 (C)). The second balloon 4 for pressing the prostate gland, as most clearly shown in Figures 1 and 3, is provided on the distal end of the catheter body 1 at the upper stream than the first balloon 3 and communicated to an outlet port 141 in the second fluid passage 14 (see Figure 2 (B)).

The distal end stopper 5 is provided on the distal end of the catheter body 1. As most clearly shown in Figure 3 and Figure 2 (D), the catheter body lis provided near on the distal end with an inlet port 152 of the urination passage 15 and an outlet port 111 of the perfusion passage 11.

A fluid (for example, distilled water, physiological saline, or the like) is poured through the one-way valve 231 in the inject and eject port 23 in the first fluid passage 13 and the one-way valve 241 in the inject and eject port 24 in the second fluid passage 14 into the first balloon 3 to be retained in the bladder and the second balloon 4 for pressing the prostate gland. Figure 4 shows the first and second balloons 3 and 4 that are inflated to the maximum volume (max. 30 cc and 3 cc, respectively). Preferably, the first balloon 3 is inflated into a substantially spherical shape while the second balloon 4 is inflated into a substantially cylindrical shape. These configurations are suitable for uniformly pressing the prostate gland as well as astriction of the prostate gland.

In order to inflate the second balloon 4 for pressing the prostate gland into a substantially cylindrical configuration, a thickness of each of the opposite ends of the second balloon 4 adapted to press the prostate gland is preferably smaller than that of an intermediate portion of the second balloon 4 (Figure 5) or the catheter body 1 is preferably provided in a plurality of longitudinal positions (three positions in the illustrated embodiment) with outlet ports 141 in the second fluid passage 14 communicated to the second balloon 4 adapted to press the prostate gland (Figure 4).

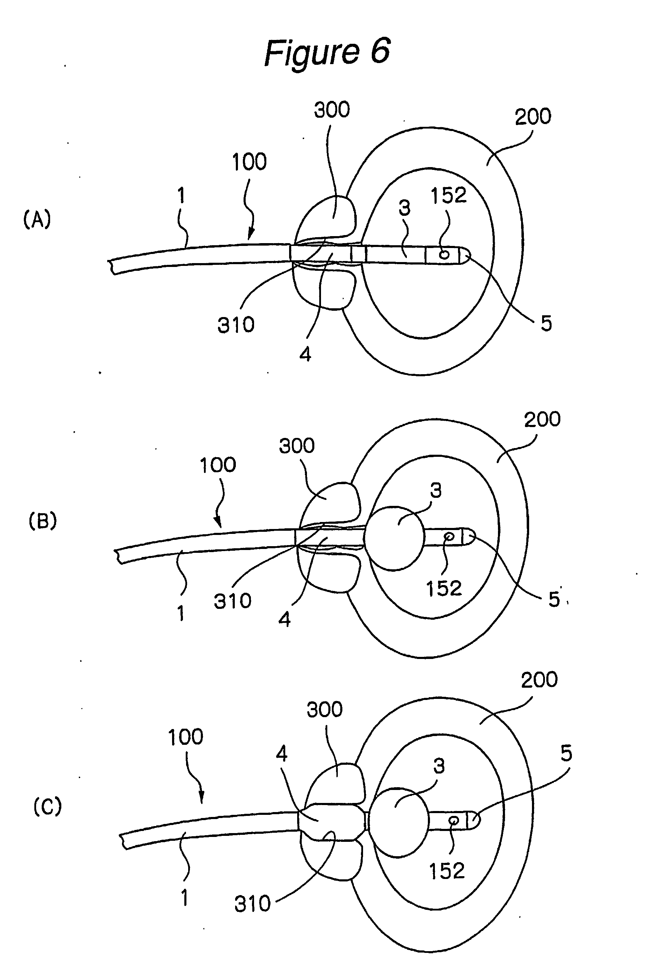

Next, referring to Figure 6, an example of utilizing the catheter for prostatomegaly treatment in accordance with the present invention in the thermotherapy for prostate gland will be described below.

As shown in Figure 6 (A), immediately after the thermotherapy is applied to a prostate gland 300, a distal end of the catheter body 1 of the catheter 100 is inserted into a bladder 200 with the first and second balloons 3 and 4 being contracted.

Then, as shown in Figure 6 (B), the first balloon 3 is inflated so that the catheter body 1 does not come out from the bladder 200 and the distal end is retained in the bladder.

Finally, as shown in Figure 6 (C), the second balloon 4 is inflated so that the balloon 4 can press prostate gland tissues 310 after being heated from the inside.

The second balloon 4 presses the heated prostate gland uniformly, enlarges atrophied area, and suppresses expansion and recovery of the prostate gland.

### [POSSIBILITY OF UTILIZATION IN AN INDUSTRY]

A catheter for prostatomegaly treatment in accordance with the present invention can be applied to a treatment for a general disease of a urinary as well as a treatment for a prostatomegaly.

## Claims

1. A catheter for prostatomegaly treatment comprises a catheter body including a perfusion passage, a first fluid passage, a second fluid passage, and an urination passage; a branch member integrally formed on a proximal end of said catheter body and having fluid injection and ejection ports communicated to said passages; a first balloon adapted to be retained in a bladder, said first balloon being provided on a distal end of the catheter body and communicated to said first fluid passage; a second balloon adapted to press a prostate gland, said second balloon being provided on a distal end of said catheter body at the upper stream than said first balloon and communicated to said second fluid passage; and a distal end stopper provided on a distal end of said catheter body.

2. A catheter for prostatomegaly treatment according to Claim 1 wherein a contrast wire is provided in said catheter body by insert molding.

3. A catheter for prostatomegaly treatment according to Claim 1 wherein a thickness of each of the opposite ends of said second balloon adapted to press the prostate gland is smaller than that of an intermediate portion of said second balloon.

4. A catheter for prostatomegaly treatment according to Claim 1 wherein said catheter body is provided in a plurality of longitudinal positions with outlet ports in said second fluid passage communicated to said second balloon adapted to press the prostate gland.
